# EUROPEAN PATENT SPECIFICATION

(11) **EP 3 419 635 B1**
(45) Date of publication and mention of the grant of the patent: **05.01.2022**
(21) Application number: 17706505.9
(22) Date of filing: 22.02.2017
(51) Int. Cl.: A61K 35/28, C12N 5/0775, A61P 29/00, A61P 19/02, A61P 27/02, A01N 1/02

(54) **PHARMACEUTICAL OR VETERINARY CELL COMPOSITIONS COMPRISING MESENCHYMAL STROMAL CELLS (MSCS) AND DIMETHYL SULFOXIDE (DMSO)**
PHARMAZEUTISCHE ODER VETERINÄRMEDIZINISCHE ZUSAMMENSETZUNGEN MIT MESENCHYMALEN STROMAZELLEN (MSC) UND DIMETHYLSULFOXID (DMSO)
COMPOSITIONS DE CELLULES PHARMACEUTIQUES OU VÉTÉRINAIRES COMPRENANT DES CELLULES STROMALES MÉSENCHYMATEUSES (CSM) ET DU SULFOXYDE DE DIMÉTHYLE (DMSO)

(30) Priority: 22.02.2016 EP 16382071
(43) Date of publication of application: 02.01.2019
(73) Proprietor: Centauri Biotech, S.L., 15142 Arteixo - A Coruna (ES)
(72) Inventor: MARIÑAS PARDO, Luis, 15142 Arteixo- A Coruña (ES); GARCÍA CASTRO, Javier, 15142 Arteixo- A Coruña (ES); RODRÍGUEZ GARCÍA, María Isabel, 15142 Arteixo- A Coruña (ES); HERMIDA PRIETO, Manuel, 15142 Arteixo- A Coruña (ES)
(74) Representative: Hoffmann Eitle
(86) International application number: PCT/EP2017/054097
(87) International publication number: WO 2017/144552

(56) References cited:
- EP-A1- 2 210 608
- WO-A1-2015/022670
- WENG JIANYU ET AL: "Mesenchymal stromal cells treatment attenuates dry eye in patients with chronic graft-versus-host disease.", MOLECULAR THERAPY : THE JOURNAL OF THE AMERICAN SOCIETY OF GENE THERAPY DEC 2012, vol. 20, no. 12, December 2012 (2012-12), pages 2347-2354, XP002769717, ISSN: 1525-0024
- KASTRINAKI MARIA-CHRISTINA ET AL: "Mesenchymal Stromal Cells in Rheumatoid Arthritis: Biological Properties and Clinical Applications", CURRENT STEM CELL RESEARCH & THERAPY, vol. 4, no. 1, January 2009 (2009-01), pages 61-69, XP002769718,
- DATABASE EMBASE [Online] ELSEVIER SCIENCE PUBLISHERS, AMSTERDAM, NL; 1 December 2015 (2015-12-01), Zakirova E Y; Nefedovskaya L V; Rizvanov A A- Valeeva A N; Faizullina R R- Akhmetshin R F: "Transplantation of allogeneic mesenchymal stromal cell for treating corneal ulcers in cats", XP002769719, Database accession no. EMB-20160254236 & ZAKIROVA E Y; NEFEDOVSKAYA L V; RIZVANOV A A- VALEEVA A N; FAIZULLINA R R- AKHMETSHIN R F: "Transplantation of allogeneic mesenchymal stromal cell for treating corneal ulcers in cats", GENES AND CELLS, vol. 10, no. 3, 1 December 2015 (2015-12-01), pages 49-55, Russia
- HIROTO MIWA ET AL: "Xeno-free proliferation of human bone marrow mesenchymal stem cells", CYTOTECHNOLOGY., vol. 64, no. 3, 15 October 2011 (2011-10-15), pages 301-308, XP055291574, NL ISSN: 0920-9069, DOI: 10.1007/s10616-011-9400-7
- DATABASE WPI Week 201573 Thomson Scientific, London, GB; AN 2015-54204T XP002760340, & CN 104 770 363 A (GUANGZHOU SALIA STEM CELL TECHNOLOGY CO) 15 July 2015 (2015-07-15)
- BROECKX SARAH ET AL: "Intravenous Application of Allogenic Peripheral Blood-Derived Mesenchymal Stem Cells: A Safety Assessment in 291 Equine Recipients", CURRENT STEM CELL RESEARCH & THERAPY, vol. 9, no. 6, 2014, pages 452-457, XP009191150,
- DATABASE WPI Week 201578 Thomson Scientific, London, GB; AN 2015-44015U XP002760342, & CN 104 630 141 A (HEILONGJIANG TIANQING STEM CELL CO LTD) 20 May 2015 (2015-05-20)
- ROY SAHELI ET AL: "A simple and serum-free protocol for cryopreservation of human umbilical cord as source of Wharton's jelly mesenchymal stem c", CRYOBIOLOGY, vol. 68, no. 3, 2014, pages 467-472, XP029030249, ISSN: 0011-2240, DOI: 10.1016/J.CRYOBIOL.2014.03.010
- AL-SAQI SHAHLA HAMZA ET AL: "Defined serum- and xeno-free cryopreservation of mesenchymal stem cells", CELL AND TISSUE BANKING, SPRINGER, NL, vol. 16, no. 2, 13 August 2014 (2014-08-13), pages 181-193, XP035500585, ISSN: 1389-9333, DOI: 10.1007/S10561-014-9463-8 [retrieved on 2014-08-13]
- DATABASE WPI Week 201224 Thomson Scientific, London, GB; AN 2011-P81794 XP002760343, & CN 102 228 472 A (WUXI RENHUAN BIOMEDICINE TECHNOLOGY CO) 2 November 2011 (2011-11-02)

## Description

### FIELD OF THE INVENTION

The present invention refers to the medical or veterinary field, in particular to the use of compositions comprising mesenchymal stromal cells (MSCs) and dimethyl sulfoxide (DMSO) in the clinical or veterinary practice.

### BACKGROUND OF THE INVENTION

Infusion of thawed products in clinical or veterinary practice has been associated with several types of adverse reactions (ARs), ranging from mild events like nausea/vomiting, hypotension or hypertension, abdominal cramps, diarrhea, flushing and chills to more severe life-threatening events like cardiac arrhythmia, encephalopathy, acute renal failure and respiratory depression. In many of these cases, these ARs have been directly attributed to dimethyl sulfoxide (DMSO). DMSO is the most commonly used cryoprotective agent (CPA), typically at a final concentration of 5 to 10% (v/v).

In fact, the physiological role of DMSO in ARs is well established. The first trial of DMSO usage for prevention of freezing damage to living cells was reported by Lovelock and Bishop in 1959 (Lovelock JE, Bishop MW. Prevention of freezing damage to living cells by dimethyl sulphoxide. Nature 1959; 183: 1394-1395). Since then DMSO has become the most widely used CPA for freezing of both cells and tissues. As part of its protective mechanism of action, DMSO can readily permeate across cell membranes to both inhibit intracellular ice formation and to prevent cell injury triggered by severe dehydration, as extracellular ice causes withdrawal of water from the intracellular milieu.

The chemical structure of DMSO ((CH₃)₂SO) results in an amphipathic molecule with one highly polar and two nonpolar domains, making it soluble in both aqueous and organic media, and thus useful for diverse laboratory and clinical purposes. DMSO is a very efficient solvent for water-insoluble compounds, a hydrogen-bond disrupter, a cell-differentiating agent, a hydroxyl radical scavenger, an intracellular low-density lipoprotein-derived cholesterol mobilizing agent and so on. It first became commercially available as a solvent in the 1950s, and following several clinical use studies in the 1960-1970s, it was approved by the United States Food and Drug Administration for the treatment of interstitial cystitis in 1978. Subsequently, DMSO has been evaluated for brain edema, amyloidosis, schizophrenia, urinary musculoskeletal and gastrointestinal disorders, pulmonary adenocarcinoma, rheumatologic and dermatologic diseases, chronic prostatitis, Alzheimer's disease and as a topical analgesic. Studies have shown that prolonged exposure to DMSO can directly impact cellular function and growth by affecting metabolism, enzymatic activity, cell cycle and apoptosis. (Aita K, Irie H, Tanuma Y, Toida S, Okuma Y, Mori S et al. Apoptosis in murine lymphoid organs following intraperitoneal administration of dimethyl sulfoxide (DMSO). Exp Mol Pathol 2005; 79: 265-271. Pal R, Bhonde R, Mamidi MK, Das AK. Diverse effects of dimethyl sulfoxide (DMSO) on the differentiation potential of human embryonic stem cells. Arch Toxicol 2012; 86: 651-661) DMSO is also thought to interfere with intracellular calcium concentration. DMSO can affect (induce or inhibit) cell apoptosis and differentiation. This effect depends on type of cell, the stage of cell development and differentiation, the specific DMSO concentration and the duration of exposure. Lin et al. (Lin C, Kalunta C, Chen F, Nguyen T, Kaptein J, Lad P. Dimethyl sulfoxide suppresses apoptosis in Burkitt's lymphoma cells. Exp Cell Res 1995; 216: 403-410) found that DMSO at concentrations higher than 1-2% could induce apoptosis in lymphoma cells. Hegner et al. (Hegner B, Weber M, Dragun D, Schulze-Lohoff E. Differential regulation of smooth muscle markers in human bone marrow-derived mesenchymal stem cells. J Hypertens 2005; 23: 1191-1202) and Ji et al. (Ji L, de Pablo J, Palecek S. Cryopreservation of adherent human embryonic stem cells. Biotechnol Bioeng 2004; 88: 299-312) found that DMSO can promote uncontrolled differentiation of stem cells. Zyuz'kov et al. (Zyuz'kov GN, Gur'yantseva LA, Simanina EV, Zhdanov VV, Dygai AM, Goldberg ED. Effect of dimethylsulfoxide on the functions of mesenchymal and hemopoietic precursors. Bull Exp Biol Med 2007; 143: 535-538) reported that DMSO can inhibit proliferation, stimulate maturation or change biological properties of the transplanted BM stem cells even when the DMSO concentration was low (0.02-0.25%). Pal et al. (Pal R, Bhonde R, Mamidi MK, Das AK. Diverse effects of dimethyl sulfoxide (DMSO) on the differentiation potential of human embryonic stem cells. Arch Toxicol 2012; 86: 651-661) studied exposure of embryoid bodies to DMSO and found effects on phenotypic characteristics, alternations in gene expression, differentiation patterns and functionality of derived hepatic cells.

All these findings imply that DMSO exposure could affect the function of cells, especially stem cells such as hematopoietic stem cells (HSC) or mesenchymal stromal cells (MSC), and influence short- and long-term engraftment ability, but it seems likely the short-term exposure and cold temperatures minimize any detrimental impact. It is also important to acknowledge that a typical 10% DMSO concentration is very hyperosmotic (2500- 3000 mOsm), and thus rapid infusion of cryopreserved cells (with 10% DMSO) into a normal isosmotic blood system can cause extreme cell volume expansion and potential osmotic injury to cells, leading directly to cell death. (Gao DY, Liu J, Liu C, McGann LE, Watson PF, Kleinhans FW et al. Prevention of osmotic injury to human spermatozoa during addition and removal of glycerol.

Hum Reprod 1995; 10: 1109-1122. Zhou X, Gao F, Shu Z, Chung J, Heimfeld S, D Gao. Theoretical and experimental analyses of optimal experimental design for determination of hydraulic conductivity of cell membrane. Biopreserv Biobank 2010; 8: 147-152.)

Thus, loss of cell viability can occur right after transfusion of stem cell-DMSO suspension, again potentially affecting engraftment. A significant uncomfortable response to injected DMSO is a garlic-like odor and taste, caused by its metabolite-dimethyl sulfide. About 45% of infused DMSO can be excreted through the urine, but a proportion of the injected DMSO is reduced to dimethyl sulfide in the body and subsequently secreted through the skin, breath, feces and urine for up to 2 days after infusion, causing the 'noxious' malodor. DMSO can also induce histamine release and can affect the central limbic-hypothalamic pathways, leading to nausea, vomiting, diarrhea, headache, flushing, fever, chills, dyspnea, anaphylaxis, vasodilatation and hypotension, pulmonary or abdominal complaints and complex reactions of cognition and emotion, and so on. (Okamoto Y, Takaue Y, Saito S, Shimizu T, Suzue T, Abe T et al. Toxicities associated with cryopreserved and thawed peripheral-blood stem-cell autografts in children with active cancer. Transfusion 1993; 33: 578-581. Ozdemir E, Akgedik K, Akdogan S, Kansu E. The lollipop with strawberry aroma may be promising in reduction of infusion-related nausea and vomiting during the infusion of cryopreserved peripheral blood stem cells. Biol Blood Marrow Transplant 2008; 14: 1425-1428. Kessinger A, Armitage JO. The evolving role of autologous peripheral stem cell transplantation following high-dose therapy for malignancies. Blood 1991; 77: 211-213.)

As such, premedication with antihistamines is typically prescribed to minimize/neutralize DMSO induced histamine release, especially in cases where it may cause some other more serious complications, such as bradycardia. DMSO, in a dose-dependent manner, has been associated with neurotoxic ARs. Hanslick et al. found that DMSO produced widespread apoptosis in the developing central nervous system. Cavaletti et al. reported that DMSO administration could induce a reduction in nerve conduction velocity and structural changes in the sciatic nerves of rats. Animal studies also showed that DMSO affected the sleep structure in rats by increasing light slow-wave sleep and reducing deep slow-wave sleep (Hanslick JL, Lau K, Noguchi KK, Olney JW, Zorumski CF, Mennerick S et al. Dimethyl sulfoxide (DMSO) produces widespread apoptosis in the developing central nervous system. Neurobiol Dis 2009; 34: 1-10. Authier N, Dupuis E, Kwasiborski A, Eschalier A, Coudore' F. Behavioural assessment of dimethylsulfoxide neurotoxicity in rats. Toxicol Lett 2002; 132: 117-121. Cavaletti G, Oggioni N, Sala F, Pezzoni G, Cavalletti E, Marmiroli P et al. Effect on the peripheral nervous system of systemically administered dimethylsulfoxide in the rat: a neurophysiological and pathological study. Toxicol Lett 2000; 118: 1-2. Topacoglu H, Karcioglu O, Ozsarac M, Oray D, Niyazi Ozucelik D, Tuncok Y. Massive intracranial hemorrhage associated with the ingestion of dimethyl sulfoxide. Vet Hum Toxicol 2004; 46: 138-140.). Similarly, DMSO can cause renal, hepatic dysfunctions and cardiovascular complications after transplantation.

In view of these studies, most investigators believe removing DMSO before infusion is beneficial. (Akkok ÇA, Holte MR, Tangen JM, Østenstad B, Bruserud Ø. Hematopoietic engraftment of dimethyl sulfoxide-depleted autologous peripheral blood progenitor cells. Transfusion 2009; 49: 354-361. Bakken AM, Bruserud O, Abrahamsen JF. No differences in colony formation of peripheral blood stem cells frozen with 5% or 10% dimethyl sulfoxide. J Hematother Stem Cell Resh 2003; 12: 351-358. Donmez A, Tombuloglu M, Gungor A, Soyer N, Saydam G, Cagirgan S. Clinical side effects during peripheral blood progenitor cell infusion. Transfus Apher Sci 2007; 36: 95-101. Otrock ZK, Beydoun A, Barada WM, Masroujeh R, Bazarbachi A, Hourani R. Transient global amnesia associated with the infusion of DMSO-cryopreserved autologous peripheral blood stem cells. Haematol Haematol 2008; 93: e36-e37. Junior AM, Arrais CA, Saboya R, Velasques RD, Junqueira PL, Dulley FL. Neurotoxicity associated with dimethylsulfoxide-preserved hematopoietic progenitor cell infusion. Bone Marrow Transplant 2008; 41: 95-96. Hentschke S, Hentschke M, Hummel K, Salwender HJ, Braumann D, Stang A. Bilateral thalamic infarction after reinfusion of DMSO-preserved autologous stem cells. Leuk Lymphoma 2006; 47: 2418-2420. Windrum P, Morris TCM, Drake MB, Niederwieser D, Ruutu T. Variation in dimethyl sulfoxide use in stem cell transplantation: a survey of EBMT centres. Bone Marrow Transplant 2005; 36: 601-603. Perseghin P, Balduzzi A, Bonanomi S, Dassi M, Buscemi F, Longoni D et al. Infusion-related side-effects in children undergoing autologous hematopoietic stem cell transplantation for acute leukemia. Bone Marrow Transplant 2000; 26: 116-118. Higman M, Port J, Beauchamp N, Chen A. Reversible leukoencephalopathy associated with re-infusion of DMSO preserved stem cells. Bone Marrow Transplant 2000; 26: 797-800. Beaujean F, Bourhis J, Bayle C, Jouault H, Divine M, Rieux C. Successful cryopreservation of purified autologous CD34+ cells: influence of freezing parameters on cell recovery and engraftment. Bone Marrow Transplant 1998; 22: 1091-1096). In addition, most DMSO depletion strategies will also concomitantly remove cell debris and reduce neutrophil, platelet and other blood cell derived soluble mediators, which may further contribute to decreasing the adverse event incidence and severity. Indeed, many studies have suggested that DMSO depletion can reduce ARs, with minimum effects or even improvements on engraftment after hematopoietic stem cells transplantation (HSCT).

Consequently, the numerous studies cited above lead the person skilled in the art away from infusing or injecting a thaw composition comprising stem cells, such as HSCs or MSC, without depleting the DMSO from said composition. Moreover, the skilled person knowing that DMSO at concentrations higher than 1-2% could induce cell apoptosis and that DMSO can inhibit proliferation, stimulate maturation or change biological properties of transplanted BM stem cells even when the DMSO concentration was as low 0.02-0.25% would departure away from using a composition comprising stem cells such as MSCs with DMSO in a v/v concentration of 10% ± 10%.

### BRIEF DESCRIPTION OF THE INVENTION

The results shown herein clearly indicate the contrary to the well-established assumptions that cell viability drastically deteriorates during the first 24 hours in culture after thawing and that Infusion of thawed cell products containing DMSO as CPA in clinical or veterinary practice is associated with several types of mild to severe adverse reactions (ARs).

In this sense, the authors of the present invention have unexpectedly found that MSCs cryopreserved by standard methods such as cell media, serum and 10% (v/v) of DMSO, do not decline drastically in viability after thawing, maintaining a significant cell viability still 24 hours afterthawing. Surprisingly as shown in example 8, these results are obtained when a basal media other than FBS (fetal bovine serum) is used, preferably when a basal media other than an animal serum media or product such as bovine serum, newborn calf serum, chicken serum, rabbit serum, goat serum, horse serum, lamb serum or porcine serum is used.

Moreover, as shown in the examples of the present invention, compositions comprising MSCs, a basal media other than FBS (fetal bovine serum) and 10% (v/v) of DMSO do not caused severe ARs.

In addition, the present disclosure shows that compositions comprising MSCs a basal media other than FBS (fetal bovine serum), preferably other than an animal serum media or product, and from 1% to 15% (v/v) of DMSO are especially useful in the treatment of a subject suffering from a degenerative joint disease such as a osteoarthritis or rheumatoid arthritis or an ocular or eye pathology such as dry eye syndrome, also known as keratoconjunctivitis, or corneal ulcer. In addition, such compositions comprising MSCs and from 1% to 15% (v/v) of DMSO are also especially useful in the treatment of an inflammatory disease, such as a disease that is accompanied by a recurrent airway obstruction such as asthma or inflammatory bowel disease (IBD) or atopic dermatitis (AD).

Therefore, a first aspect of the invention refers to a pharmaceutical composition which comprises:
a. a population of mesenchymal stromal cells (MSCs) in which at least 50% of said population by number of cells are MSCs;
b. the basal media Dulbecco's Modified Eagle's medium (DMEM); and
c. dimethyl sulfoxide (DMSO) in a v/v concentration (DMSO volume percentage over the final volume of the nutrient rich liquid prepared for cell culture) of 10% ± 10%;
for use in the treatment of a subject suffering from osteoarthritis; wherein said composition is suitable for local administration and is administered via the intraarticular route.

In a preferred embodiment of the first aspect of the invention, the mesenchymal stromal cells are umbilical cord-derived stromal cells, adipose tissue-derived stromal cells, expanded mesenchymal stromal cells, expanded adipose tissue-derived stromal cells, bone-marrow derived stromal cells or expanded bone-marrow derived stromal cells.

In another preferred embodiment of the first aspect of the invention or of any of its preferred embodiments, the MSCs are allogeneic or autologous.

In another preferred embodiment of the first aspect of the invention or of any of its preferred embodiments, the composition preferably comprises between about 0.25 x 10⁶ cells/kg to about 5 x 10⁶ cells/kg of subject weight. Such composition preferably comprises a volume of the nutrient rich liquid prepared for cell culture, DMEM, of about 0.5 y 3 ml, preferably about 2 ml. More preferably such composition is within a pre-filled syringe, more preferably within a tube or vial, still more preferably within a cryotube or a cryovial. More preferably such composition is diluted in a total volume of from 200 to 250 ml of a saline or sugar (glucose) solution.

In another preferred embodiment of the first aspect of the invention or of any of its preferred embodiments, the composition preferably comprises between about 1.0 x 10⁶ cells to about 20 x 10⁶ cells. Such composition preferably comprises a volume of the nutrient rich liquid prepared for cell culture, DMEM, of about 0.5 to 3 ml, preferably about 2 ml. More preferably such composition is within a pre-filled syringe, more preferably within a tube or vial, still more preferably within a cryotube or a cryovial.

In another preferred embodiment, the subject is a non-human mammal such as a dog or a horse or a cat.

### BRIEF DESCRIPTION OF THE FIGURES

**FIG 1.** A) Ultrasound images tendon pre-treatment of the horse of example 3. B) Ultrasound images tendon after treatment (35 days) of the horse of example 3.

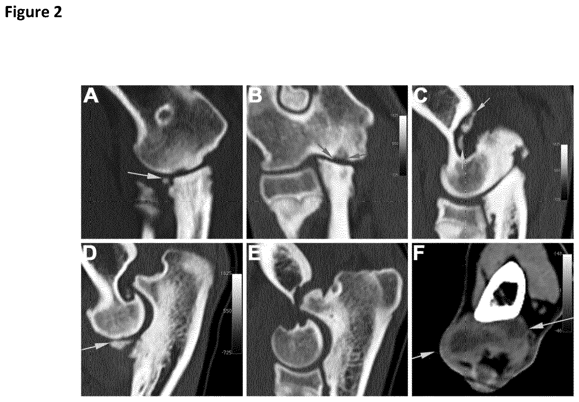

**FIG 2****.**_This figure represents the dog suffering osteoarthritis secondary to Elbow Dysplasia referred to in example 5. This figure shows a computer aided Tomography in which the left elbow (A - C) had a bone fragment (6x4x2mm, image A). Big size defect could also be seen (Image B), and several osteophytes in the articular margin (imagen C). On the left elbow (D - E) analogue changes could be seen but even more severe, with articular incongruence and more advanced osteoarthritis. Severe synovial effusion is shown in image F.
**FIG 3****.** This figure is a CT scan which shows a fragmented coronoid process and humeral cartilage injury in both elbows (A left and B right), thickened synovial capsules in both shoulders (C left and D right) and also hip dysplasia with edema (E). This figure refers to the dog of example 6.

### DETAILED DESCRIPTION OF THE INVENTION

### DEFINITIONS

As used herein, the following terms and phrases shall have the meanings set forth below. Unless defined otherwise, all technical and scientific terms used herein have the same meaning as commonly understood to one of ordinary skill in the art to which this invention belongs.

The articles "a" and "an" refer to one or to more than one (i.e., to at least one) of the grammatical object of the article. By way of example, "an element" means one element or more than one element.

The term "about" when used in relation to a value relates to the value ±10%.

By "adipose tissue" is meant any fat tissue. The adipose tissue may be brown or white adipose tissue, derived from, for example, subcutaneous, omental/visceral, mammary, gonadal, or other adipose tissue site. Preferably, the adipose tissue is subcutaneous white adipose tissue. The adipose tissue may comprise a primary cell culture or an immortalized cell line. The adipose tissue may be from any organism having fat tissue. In some embodiments, the adipose tissue is mammalian, and in further embodiments the adipose tissue is human. A convenient source of adipose tissue is liposuction surgery. However, it will be understood that neither the source of adipose tissue nor the method of isolation of adipose tissue is critical to the invention. If cells as described herein are desired for autologous transplantation into a subject, the adipose tissue will be isolated from that subject.

"Adipose tissue-derived stromal cells (ASCs)" refers to MSCs that originate from adipose tissue, generally from human adipose tissue (hASCs). These cells have been used in the present invention to reproduce the invention.

The term "cells/kg" as used herein shall be taken to mean the number of cells (e.g. MSC) administered per kilogram of patient body weight.

The term "culture" refers to the growth of cells, organisms, multicellular entities, or tissue in a medium. The term "culturing" refers to any method of achieving such growth, and may comprise multiple steps. The term "further culturing" refers to culturing a cell, organism, multicellular entity, or tissue to a certain stage of growth, then using another culturing method to bring said cell, organism, multicellular entity, or tissue to another stage of growth. A "cell culture" refers to a growth of cells in vitro. In such a culture, the cells proliferate, but they do not organize into tissue per se. A "tissue culture" refers to the maintenance or growth of tissue, e.g., explants of organ primordial or of an adult organ in vitro so as to preserve its architecture and function. A "monolayer culture" refers to a culture in which cells multiply in a suitable medium while mainly attached to each other and to a substrate. Furthermore, a "suspension culture" refers to a culture in which cells multiply while suspended in a suitable medium. Likewise, a "continuous flow culture" refers to the cultivation of cells or explants in a continuous flow of fresh medium to maintain cell growth, e.g. viability. The term "conditioned media" refers to the supernatant, e.g. free of the cultured cells/tissue, resulting after a period of time in contact with the cultured cells such that the media has been altered to include certain paracrine and/or autocrine factors produced by the cells and secreted into the culture. A "confluent culture" is a cell culture in which all the cells are in contact and thus the entire surface of the culture vessel is covered, and implies that the cells have also reached their maximum density, though confluence does not necessarily mean that division will cease or that the population will not increase in size.

The term "culture medium" or "medium" is recognized in the art, and refers generally to any substance or preparation used for the cultivation of living cells. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. Exemplary gaseous media include the gaseous phase that cells growing on a petri dish or other solid or semisolid support are exposed to. The term "medium" also refers to material that is intended for use in a cell culture, even if it has not yet been contacted with cells. In other words, a nutrient rich liquid prepared for bacterial culture is a medium. Similarly, a powder mixture that when mixed with water or other liquid becomes suitable for cell culture may be termed a "powdered medium". "Defined medium" refers to media that are made of chemically defined (usually purified) components. "Defined media" do not contain poorly characterized biological extracts such as yeast extract and beef broth. "Rich medium" includes media that are designed to support growth of most or all viable forms of a particular species. Rich media often include complex biological extracts. The term "basal medium" refers to a medium which promotes the growth of many types of microorganisms which do not require any special nutrient supplements. Most basal media generally comprise four basic chemical groups: amino acids, carbohydrates, inorganic salts, and vitamins. A basal medium generally serves as the basis for a more complex medium, to which supplements such as serum, buffers, growth factors, lipids, and the like are added. Examples of basal media include, but are not limited to, Eagles Basal Medium, Minimum Essential Medium, Dulbecco 's Modified Eagle's Medium, Medium 199, Nutrient Mixtures Ham's F-10 and Ham's F-12, McCoy's 5 A, Dulbecco's MEM/F-I 2, RPMI 1640, and Iscove's Modified Dulbecco's Medium. It is noted that many modifications of Eagle's Medium have been developed since the original formulation appeared in the literature. Among the most widely used of these modifications is Dulbecco's Modified Eagle's medium (DMEM). DMEM is a modification of Basal Medium Eagle (BME) that contains a four-fold higher concentration of amino acids and vitamins, as well as additional supplementary components. The original DMEM formula contains 1000 mg/L of glucose and was first reported for culturing embryonic mouse cells (low glucose DMEM). A further alteration with 4500 mg/L glucose has proved to be optimal for cultivation of certain cell types (high glucose DMEM). DMEM is the basal media in the context of the present invention. It is noted that the basal media referred to in the present invention is an animal/human serum-free media or a chemically defined serum-free and xeno free media designed to grow MSCs being Dulbecco's Modified Eagle's Medium (DMEM). It is noted that the most commonly used animal serum supplement is FBS and fetal calf serum, these animal serum, as well as the human serum, are excluded from the pharmaceutical or veterinary composition of the invention.

In the context of the present invention, an animal/human serum-free media or a chemically defined animal/human serum-free and xeno free media is understood as a media that contains no animal or human-derived blood or serum components. In particular, it refers to a media that contains no human or animal proteins present in the blood of humans or animals such as albumin or fetal bovine serum or fetal calf serum. An example of an animal/human serum-free media or a chemically defined animal/human serum-free and xeno free media is DMEM.

The terms "comprise" and "comprising" are used in the inclusive, open sense, meaning that additional elements may be included.

The term "expanded" as used herein when referring to cells shall be taken to have its usual meaning in the art, namely cells that have been proliferated in vitro. A MSC can be expanded to provide a population of cells that retain at least one biological function of the MSC, typically the ability to adhere to a plastic surface, under standard culture conditions. The expanded population of cells may retain the ability to differentiate into one or more cell types.

The term "including" is used herein to mean "including but not limited to". "Including" and "including but not limited to" are used interchangeably.

"Mesenchymal stromal cells (also referred to herein as "MSCs") are multipotent stromal cells, i.e. they are cells which are capable of giving rise to multiple different types of cells. These types of cells are defined by their plastic adherent growth and subsequent expansion under specific culture conditions and by their in vitro and in vivo differentiation potential (Caplan Al: Mesenchymal Stem Cells. J Orthop Res 1991, 9:641-650; Zuk PA, Zhu M, Mizuno H, Huang J, Futrell JW, Katz AJ, Benhaim AJ, Lorenz HP, Hedrick MH: Multi-lineage cells from human adipose tissue: implication for cell- based therapies. Tissue Eng 2001, 7:211-228; Huang JI, Beanes SR, Zhu M, Lorenz HP, Hedrick MH, Benhaim P: Rat extramedullary adipose tissue as a source of osteochondrogenic progenitor cells. Plast Reconstr Surg 2002, 109:1033-1041; Dennis JE, Caplan Al: Differentiation potential of conditionally immortalized mesenchymal progenitor cells from adult marrow of a H-2Kb-tsA58 transgenic mouse. J Cell Physiol 1996, 167(3):523-538; Johnstone B, Hering TM, Caplan Al, Goldberg VM, Yoo JU: In vitro chondrogenesis of bone marrow-derived mesenchymal progenitor cells. Exp Cell Res 1998, 238(1):265-272; Dennis JE, Merriam A, Awadallah A, Yoo JU, Johnstone B, Caplan Al: A quadr- icpotential mesenchymal progenitor cell isolated from the marrow of an adult mouse. J Bone Miner Res 1999, 14(5):700-709; Kopen GC, Prockop DJ, Phinney DG: Marrow stromal cells migrate throughout forebrain and cerebellum, and they differentiate into astrocytes after injection into neonatal mouse brains. Proc Natl Acad Sci USA 1999, 96:10711-10716; Toma C, Pittenger MF, Cahill KS, Byrne BJ, Kessler PD: Human mesenchymal stem cells differentiate to a cardiomyocyte phenotype in the adult murine heart. Circulation 2002, 105:93-98). MSCs are well known in the literature as shown above and as such form part of the common general knowledge attributable to the skilled person in the art.

MSCs have been isolated from numerous mammals. For example, MSCs have been derived from various equine tissues, including bone marrow (Arnhold SJ, Goletz I, Klein H, Stumpf G, Beluche LA, Rohde C, Addicks K, Litzke LF. Isolation and characterization of bone marrow-derived equine mesenchymal stem cells. Am J Vet Res 2007, 68, 1095-1105; Kisiday JD, Kopesky PW, Evans CH, Grodzinsky AJ, Mcllwraith CW, Frisbie DD. Evaluation of adult equine bone marrow- and adipose-derived progenitor cell chondrogenesis in hydrogel cultures. J Orthop Res 2008, 26, 322-33; Smith RKW, Korda M, Blunn GW, Goodship AE. Isolation and implantation of autologous equine mesenchymal stem cells from bone marrow into the superficial digital flexor tendon as a potential novel treatment. Equine Vet J 2003, 35, 99-102), adipose tissue (Vidal MA, Kilroy GE, Johnson JR, Lopez MJ, Moore RM, Gimble JM. Cell growth characteristics and differentiation frequency of adherent equine bone marrow-derived mesenchymal stromal cells: adipogenic and osteogenic capacity. Vet Surg 2006, 35, 601-610.), peripheral blood, umbilical cord blood, umbilical cord matrix, and amniotic fluid.

In addition, biological characterization of canine MSCs isolated from bone marrow and adipose tissue is well documented in Hiroshi Takemitsu et al. Comparison of bone marrow and adipose tissue-derived canine mesenchymal stem cells. BMC Veterinary Research 2012, 8:150. Lastly, MSCs have been successfully isolated from bone marrow and adipose tissue (Caplan Al: Mesenchymal Stem Cells. J Orthop Res 1991, 9:641-650. Zuk PA, Zhu M, Mizuno H, Huang J, Futrell JW, Katz AJ, Benhaim AJ, Lorenz HP, Hedrick MH: Multi-lineage cells from human adipose tissue: implication for cell- based therapies. Tissue Eng 2001, 7:211-228. Huang JI, Beanes SR, Zhu M, Lorenz HP, Hedrick MH, Benhaim P: Rat extramedullary adipose tissue as a source of osteochondrogenic progenitor cells. Plast Reconstr Surg 2002, 109:1033-1041.) in humans.

A "patient", "subject" or "host" to be treated by the subject method may mean either a human or non- human animal. Preferably in the present invention is a non-human animal such as a dog or a horse.

The term "pharmaceutical composition" refers to a composition intended for use in therapy, no matter if it is for human or animal therapy, so that within the term pharmaceutical composition of the present invention veterinary compositions are encompassed. The compositions of the invention are pharmaceutical compositions, intended for use in the treatment of a patient or host. The compositions of the invention must include, in addition to MSCs, a nutrient rich liquid prepared for cell culture, being DMEM, and dimethyl sulfoxide (DMSO) in a v/v concentration of about 10%. In addition the pharmaceutical composition may include non-cellular components (from hereinafter this composition is herein referred to as "the pharmaceutical composition of the invention"). It is important to note that the nutrient rich liquid prepared for cell culture, preferably a basal media, is does not comprise FBS (fetal bovine serum) or any human/animal serum.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient.

As used herein, the term "solution" includes a pharmaceutically acceptable carrier or diluent in which the MSCs used in the invention remain viable.

The term "substantially pure", with respect to MSC populations, refers to a population of cells in which at least about 50%, 60%, 70%, 75%, at least about 85%, at least about 90%, or at least about 95%, by number of the cells are MSCs. In other words, the term "substantially pure", with respect to MSC populations, refers to a population of cells that contains less than about 50%, less than about 30%, less than about 20%, less than about 10%, or less than about 5%, by number of lineage committed cells. "Support" as used herein refers to any device or material that may serve as a foundation or matrix for the growth of adipose tissue-derived stromal cells.

The term "dimethyl sulfoxide (DMSO) is an organosulfur compound with the formula (CH₃)₂SO. This colorless liquid is an important polar aprotic solvent that dissolves both polar and nonpolar compounds and is miscible in a wide range of organic solvents as well as water. It has a relatively high melting point. DMSO has the unusual property that many individuals perceive a garlic-like taste in the mouth after contact with the skin. In terms of chemical structure, the molecule has idealized Cs symmetry. It has a trigonal pyramidal molecular geometry consistent with other three-coordinate S(IV) compounds, with a nonbonded electron pair on the approximately tetrahedral sulfur atom.

### DETAILED DESCRIPTION

A first aspect of the invention refers to a pharmaceutical composition which comprises:
a. a population of mesenchymal stromal cells (MSCs) in which at least 50% of said population by number of cells are MSCs;
b. the basal media Dulbecco's Modified Eagle's medium (DMEM); and
c. dimethyl sulfoxide (DMSO) in a v/v concentration (DMSO volume percentage over the final volume of the nutrient rich liquid prepared for cell culture) of 10%±10%;
for use in the treatment of a subject suffering from osteoarthritis; wherein said composition is suitable for local administration and is administered via the intraarticular route.

It is noted that the percentage by volume of the DMSO is calculated over the total final volume of the nutrient rich liquid prepared for cell culture present in the final composition.

### THE MSCs OF THE FIRST ASPECT OF THE INVENTION

The MSCs of the first aspect of the invention are undifferentiated stromal cells having the capacity to differentiate to other cells, and are typically derived from connective tissue, and are thus non-hematopoietic cells. The term "connective tissue" refers to tissue derived from mesenchyme and includes several tissues which are characterized in that their cells are included within the extracellular matrix. Among the different types of connective tissues, adipose and cartilaginous tissues are included. In one embodiment, the MSCs are from the stromal fraction of the adipose tissue. In another embodiment, the MSCs are obtained from chondrocytes e.g. from hyaline cartilage. In another embodiment, the MSCs are obtained from skin. Also, in another embodiment, the MSCs are obtained from bone marrow. Alternative sources of MSCs include but are not limited to periosteum, dental pulp, spleen, pancreas, ligament, tendon, skeletal muscle, umbilical cord and placenta. Other sources are induced pluripotent stem cells.

The MSCs can be obtained from any suitable source and from any suitable animal, including humans. Typically, said cells are obtained from post-natal mammalian connective tissues. In a preferred embodiment, the MSCs are obtained from a source of connective tissue, such as the stromal fraction of adipose tissue, hyaline cartilage, bone marrow, skin etc. Also, in a particular embodiment, the MSCs are from a mammal, e.g., a rodent, primate, etc., preferably, from a horse or a dog. Typically, the MSCs are obtained from the stromal fraction of human adipose tissue, i.e. they are adipose tissue- derived stromal cells (ASCs).

The MSCs are adherent to plastic under standard culture conditions. MSCs are undifferentiated multipotent cells, having the capacity to differentiate into or towards somatic cells such as mesodermal cells (e.g. adipose, chondrocytes, osteoblasts) and optionally into or towards endodermal and/or ectodermal cell types or lineages. Typically, the cells have the capacity to differentiate into or towards at least two or all cell types selected from the group consisting of adipocytic, chondroblastic and osteoblastic lineages.

In one embodiment of the first aspect of the invention, the MSCs are in vitro culture expanded MSCs or the in vitro culture expanded progeny thereof (hereinafter both are referred to as expanded MSCs or "eMSCs"). Methods for the preparation of eMSCs are known in the art, for example as described in WO2007039150. eMSCs retain several phenotypic characteristics of MSC, e.g. the eMSCs are adherent to plastic under standard culture conditions and retain an undifferentiated phenotype.

eMSCs are undifferentiated multipotent cells, having the capacity to differentiate into somatic cells such as mesodermal cells. Whereas MSCs have the capacity to differentiate towards at least one or more specialized cell lineages such as but not limited to adipocyte, chondroblastic and osteoblastic lineages; typically, in eMSCs this capacity to differentiate is reduced or may even be absent e.g. whereas a MSCs may differentiate towards at least the osteogenic and adipocytic lineages, the eMSCs derived therefrom may differentiate only towards the adipocytic lineage. This may be advantageous for therapeutic applications of the cells, where the cells may be administered to patients as it can reasonably be expected that unanticipated and potentially harmful differentiation of eMSCs will be less likely to occur.

In one embodiment the eMSCs may be the progeny of stem cells. Typically, the eMSCs (i) do not express markers specific from APCs; (ii) do not express IDO constitutively (iii) do not significantly express MHC II constitutively. Typically, expression of IDO or MHC II may be induced by stimulation with IFN-y.

### THE CELL POPULATION OF THE FIRST ASPECT OF THE INVENTION

The cell population referred to in the first aspect of the invention is also referred to as "cell populations of the invention". Typically, the MSCs are expanded ASCs, typically allogeneic or autologous expanded ASCs. Typically the cell populations of the invention are a homogenous or substantially homogenous population of MSCs and/or eMSCs. Cell populations of the invention comprise or comprise essentially of MSCs and/or eMSCs, however cell populations of the invention may also comprise other cell types. Accordingly in one embodiment the invention provides cell populations of the invention in which at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells are MSCs and/or eMSCs.

Typically the cell populations of the invention are a culture expanded population of MSCs, comprising or comprising essentially of eMSCs, however cell populations of the invention may also comprise other cell types. Accordingly in one embodiment the invention provides cell populations of the invention in which at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells are eMSCs. In one embodiment, the eMSCs are eASCs, for example human eASCs. In a particular embodiment, the cells are allogeneic with respect to the subject to be treated. In another particular embodiment, the cells are autologous with respect to the subject to be treated.

Typically, a cell population of the invention may have the capacity to differentiate towards at least one or more specialized cell lineages such as but not limited to adipocytic, chondroblastic and osteoblastic lineages. In one embodiment a cell population of the invention may have the capacity to differentiate into or towards at least two or all cell types selected from the group consisting of adipocytic, chondroblastic and osteoblastic lineages. However, in an alternative embodiment this capacity to differentiate is reduced or may even be absent e.g. whereas a MSC may differentiate towards at least the osteogenic and adipocytic lineages, the eMSC population derived therefrom may differentiate only towards the adipocytic lineage. This may be advantageous for therapeutic applications of the cells, where the cells may be administered to patients as it can reasonably be expected that unanticipated and potentially harmful differentiation of eMSCs will be less likely to occur.

In some embodiments MSCs or eMSCs are pre-stimulated in order to enhance one or more of their proliferation capacity, migration capacity, survival capacity, therapeutic effect and immunoregulatory properties. Typically, at least about 40% (e.g. at least about 50%, at least about 60%, at least about 70%, at least about 80%, at least about 85%, at least about 90%, at least about 95% at least about 96%, at least about 97%, at least about 98%, or at least about 99%) of the cell populations of the invention are pre-stimulated in order to enhance one or more of their proliferation capacity, migration capacity, survival capacity, therapeutic effect and immunoregulatory properties. In some embodiments, pre-stimulation may be achieved by contacting the MSCs with a cytokine. In some embodiments of the invention, pre-stimulation may be achieved by contacting the MSCs with IFN-y.

### THE PHARMACEUTICAL OR VETERINARY COMPOSITION OF THE FIRST ASPECT OF THE INVENTION

The pharmaceutical or veterinary composition of the invention must include, in addition to MSCs as defined above, a nutrient rich liquid prepared for cell culture, preferably other than FBS, and dimethyl sulfoxide (DMSO) in a v/v (volume per final volume of basal media) concentration of about 10%. Surprisingly as shown in the examples, compositions comprising MSCs, a basal media other than FBS (fetal bovine serum) and about 10% (v/v) of DMSO do not caused severe ARs.

Such composition of the invention may include a substantially pure population of MSCs or eMSCs, for example a substantially pure population of ASCs or eASCs, e.g. human eASCs. The MSCs, eMSCs, ASCs or eASCs may be stem cells. The composition of the present invention may also include cell culture components, e.g., culture media including one or more of amino acids, metals and coenzyme factors. The composition may include small populations of other stromal cells. The composition may also include other non-cellular components which may support the growth and survival of the MSCs under particular circumstances, e.g. implantation, growth in continuous culture, or use as a biomaterial or composition.

The pharmaceutical or veterinary composition of the invention will usually contain as final basal media DMEM, volume of between 0.5 and 3 ml and a cell concentration of between 1X10⁶ and 20X10⁶ cells in such volume.

The pharmaceutical or veterinary composition of the invention will comprise a nutrient rich liquid prepared for cell culture, a basal media. The compositions of the invention comprise DMEM as the carrier, which may optionally be supplemented at a concentration of e.g. up to about 5%, up to about 10%, up to about 15%, up to about 20%, up to about 25%, up to about 30%. Examples of additional carriers and diluents which could be used in the composition of the invention are well known in the art, and may include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

The pharmaceutical or veterinary composition of the invention may be sterile and/or fluid to the extent that easy syringability exists. In addition, the composition may be stable under the conditions of manufacture and storage, and/or preserved against the contaminating action of microorganisms such as bacteria and fungi through the use of, for example, parabens, chlorobutanol, phenol, ascorbic acid and thimerosal.

### INDICATIONS OF THE PHARMACEUTICAL OR VETERINARY COMPOSITION OF THE INVENTION.

The present invention provides uses of the pharmaceutical or veterinary composition of the invention in the treatment of inflammatory joint diseases or degenerative joint diseases, in particular osteoarthritis.

Typically, the pharmaceutical or veterinary composition of the invention is used in treating diseases such as inflammatory joint diseases or degenerative joint diseases in patients during an acute phase of the disease. The term "acute phase" shall be taken to mean a patient experiencing significant inflammation of one or more joints, as opposed to only mild or moderate inflammation. The acute phase is also referred to as "flares" in the art.

Typically, the patient can receive the pharmaceutical or veterinary composition of the invention for at least about 6, 12, 18, 24 or 36 weeks and still presents symptoms of active disease. In other words, even after treatment for at least about 6, 12, 18, 24 or 36 weeks, the severity of the disease is not ameliorated.

Said therapies may include one or more of disease-modifying antirheumatic drugs (DMARDs), nonsteroidal anti-inflammatories (NSAIDs), biological medicinal products and corticosteroids. NSAIDs including but are not limited to Cox-2 inhibitors, diclofenac, ibuprofen, naproxen, celecoxib, mefenamic acid, etoricoxib, indometacin and aspirin. Corticosteroids include but are not limited to triamcinolone, cortisone, prednisone, and methylprednisolone. Disease-modifying antirheumatic drugs (DMARDs), include but are not limited to auranofin, choroquine, cyclosporine, cyclophosphamide, gold preparations, hydroxychloroquine sulphate, leflunomide, methotrexate, penicillamine, sodium aurothiomalate and sulfasalazine.

In one embodiment the patient is treatment refractory to at least 2 or 3 DMARDs, preferably selected from the group consisting of hydroxychloroquine, leflunomide, methotrexate, minocycline, and sulfasalazine (e.g., methotrexate and hydroxychloroquine; methotrexate and leflunomide; methotrexate and sulfasalazine; sulfasalazine and hydroxychloroquine; methotrexate, hydroxychloroquine and sulfasalazine).

These drugs are targeted to specific anti-inflammatory molecular pathways. In contrast, MSCs provide a more physiological approach, which involves modulating multiple inflammatory pathways and providing a global dampening of pathological hyper-inflammatory responses. The MSCs also provide a mechanism for negative feedback loops which would enable a physiological balance of pro- and anti-inflammatory cytokines to be struck, whereas this is not possible with a mere delivery of predetermined doses of anti-inflammatory drugs. Thus, MSCs of the invention are particularly effective in treating any inflammatory disease in patients who are treatment refractory to any of the anti-inflammatory drugs. In one embodiment it is particularly preferred that the patient is treatment refractory to at least methotrexate. In one embodiment the patient is treatment refractory to at least 2 or 3 DMARDs, comprising methotrexate and one or more DMARDs selected from the group consisting of hydroxychloroquine, leflunomide, minocycline, and sulfasalazine.

The examples presented herein show that inflammatory diseases can be treated by administering ASCs, and that the therapeutic effect is particularly good when ASCs are first administered at early/moderate stages of the disease. Surprisingly, beneficial effects such as long-term therapeutic effects are observed even after treatment with ASCs is discontinued as shown in the examples. These additional effects are associated to the DMSO contained in the pharmaceutical or veterinary composition of the invention.

### ADMINISTRATION OF THE PHARMACEUTICAL OR VETERINARY COMPOSITION OF THE INVENTION.

In one embodiment, the pharmaceutical or veterinary composition of the invention is prepared for local administration and administered by the intra articular route. In this embodiment, the MSCs can be ASCs, for example eASCs.

In one embodiment, the MSCs used in the invention may be autologous with respect to the subject to be treated. In a further embodiment, the MSCs used in the invention may be allogeneic or xenogeneic with respect to the subject to be treated. Allogenic MSCs derived from a donor may theoretically be used for the treatment of any patient, irrespective of MHC incompatibility. In one embodiment, the composition of the invention may be administered by injection or implantation of the composition at one or more target sites in the subject to be treated. In a further embodiment, the composition of the invention may be inserted into a delivery device which facilitates introduction of the composition into the subject by injection or implantation. In one embodiment the delivery device may comprise a catheter. In a further embodiment, the delivery device may comprise a syringe.

### DOSAGE OF THE PHARMACEUTICAL OR VETERINARY COMPOSITION OF THE INVENTION.

The dosage of MSCs and any further therapeutic agent will vary depending on the symptoms, age and body weight of the patient, the nature and severity of the disorder to be treated or prevented, the route of administration, and the form of the further therapeutic agent. The compositions of the invention may be administered in a single dose or in divided doses.

Appropriate dosages for MSCs and any further therapeutic agent(s) may be determined by known techniques.

Typically said dose is about 10 x 10⁶ cells/kg of subject weight or lower, is about 9 x 10⁶ cells/kg or lower, is about 8 x 10⁶ cells/kg or lower, is about 7 x 10⁶ cells/kg or lower, is about 6 x 10⁶ cells/kg or lower, is about 5 x 10⁶cells/kg or lower. In an alternative embodiment said dose may be between about 0.25 x 10⁶ cells/kg to about 5 x 10⁶cells/kg ; or more preferably about 1 x 10⁶ cells/kg to about 5 x 10⁶ cells/kg. Accordingly in further alternative embodiments the dose may be about 0.25 x 10⁶ cells/kg, 0.5 x 10⁶ cells/kg, 0.6 x 10⁶ cells/kg, 0.7 x 10⁶ cells/kg; 0.8 x 10⁶ cells/kg; 0.9 x 10⁶cells/kg; 1.1 x 10⁶cells/kg; 1.2 x 10⁶cells/kg; 1.3 x 10⁶cells/kg; 1.4 x 10⁶cells/kg; 1.5 x 10⁶ cells/kg; 1.6 x 10⁶ cells/kg; 1.7 x 10⁶ cells/kg; 1.8 x 10⁶ cells/kg; 1.9 x 10⁶ cells/kg or 2 x 10⁶ cells/kg. The dose may, in other embodiments, be between 0.1 and 1 million cells / kg; or between 1 and 2 million cells / kg; or between 2 and 3 million cells / kg; or between 3 and 4 million cells / kg; or between 4 and 5 million cells / kg; or between 5 and 6 million cells / kg; or between 6 and 7 million cells / kg; or between 7 and 8 million cells / kg; or between 8 and 9 million cells / kg; or between 9 and 10 million cells / kg.

In an alternative embodiment the cells may be administered to the patient as a fixed dose, independent of patient weight. Typically said dose is between about 10 million cells and 500 million cells, e.g. said dose is about 10 x 10⁶cells, 50 x 10⁶cells, 10 x 10⁶cells, 50 x 10⁶ cells.

The pharmaceutical or veterinary composition of the invention when used for local administration will usually contain a final basal media, DMEM, volume of between 0.5 and 3 ml and a cell concentration of between 1X10⁶ and 20X10⁶ cells in such volume. The pharmaceutical or veterinary composition of the invention when used for systemic administration will usually contain a final basal media, DMEM, volume of between 0.5 and 3 ml and comprised between about 0.25 x 10⁶ cells/kg to about 5 x 10⁶ cells/kg of subject weight.

The precise time of administration and amount of any particular agent that will yield the most effective treatment in a given patient will depend upon the activity, pharmacokinetics, and bioavailability of the agent, the physiological condition of the patient (including age, sex, disease type and stage, general physical condition, responsiveness to a given dosage and type of medication), the route of administration, etc.. The information presented herein may be used to optimize the treatment, e.g., determining the optimum time and/or amount of administration, which will require no more than routine experimentation, such as monitoring the subject and adjusting the dosage and/or timing. While the subject is being treated, the health of the subject may be monitored by measuring one or more of relevant indices at predetermined times during a 24 hour period. Treatment regimens, including dosages, times of administration and formulations, may be optimized according to the results of such monitoring.

Treatment may be initiated with smaller dosages which are less than the optimum dose. Thereafter, the dosage may be increased by small increments until the optimum therapeutic effect is attained.

The combined use of several therapeutic agents may reduce the required dosage for any individual component because the onset and duration of effect of the different components may be complimentary. In such combined therapy, the different active agents may be delivered together or separately, and simultaneously or at different times within the day.

### FURTHER THERAPEUTIC AGENTS OF THE PHARMACEUTICAL OR VETERINARY COMPOSITION OF THE INVENTION.

In one embodiment, the pharmaceutical or veterinary composition of the invention may contain or alternatively may be administered in conjunction with one or more (or two or more, or three or more, e.g. 1, 2, 3, 4 or 5) further therapeutic agents.

In some embodiments, the MSCs and the one or more further therapeutic agents may be administered to the subject simultaneously. In other embodiments, the MSCs and the one or more further therapeutic agents may be administered to the subject sequentially. The one or more further therapeutic agents may be administered before or after administration of the MSCs.

Said further therapeutic agent may be selected from the following: an analgesic, such as a nonsteroidal anti-inflammatory drug, an opiate agonist or a salicylate; an anti-infective agent, such as an antihelmintic, an antianaerobic, an antibiotic, an aminoglycoside antibiotic, an antifungal antibiotic, a cephalosporin antibiotic, a macrolide antibiotic, a β-lactam antibiotic, a penicillin antibiotic, a quinolone antibiotic, a sulfonamide antibiotic, a tetracycline antibiotic, an antimycobacterial, an antituberculosis antimycobacterial, an antiprotozoal, an antimalarial antiprotozoal, an antiviral agent, an anti-retroviral agent, a scabicide, an anti-inflammatory agent, a corticosteroid anti-inflammatory agent, an antipruritics/local anesthetic, a topical anti-infective, an antifungal topical anti-infective, an antiviral topical anti-infective; an electrolytic and renal agent, such as an acidifying agent, an alkalinizing agent, a diuretic, a carbonic anhydrase inhibitor diuretic, a loop diuretic, an osmotic diuretic, a potassium-sparing diuretic, a thiazide diuretic, an electrolyte replacement, and an uricosuric agent; an enzyme, such as a pancreatic enzyme and a thrombolytic enzyme; a gastrointestinal agent, such as an antidiarrheal, an antiemetic, a gastrointestinal antiinflammatory agent, a salicylate gastrointestinal antiinflammatory agent, an antacid anti-ulcer agent, a gastric acid-pump inhibitor anti-ulcer agent, a gastric mucosal anti-ulcer agent, a H2 -blocker antiulcer agent, a cholelitholytic agent, a digestant, an emetic, a laxative and stool softener, and a prokinetic agent; a general anesthetic, such as an inhalation anesthetic, a halogenated inhalation anesthetic, an intravenous anesthetic, a barbiturate intravenous anesthetic, a benzodiazepine intravenous anesthetic, and an opiate agonist intravenous anesthetic; a hormone or hormone modifier, such as an abortifacient, an adrenal agent, a corticosteroid adrenal agent, an androgen, an anti- androgen, an immunobiologic agent, such as an immunoglobulin, an immunosuppressive, a toxoid, and a vaccine; a local anesthetic, such as an amide local anesthetic and an ester local anesthetic; a musculoskeletal agent, such as an anti-gout anti-inflammatory agent, a corticosteroid antiinflammatory agent, a gold compound anti-inflammatory agent, an immunosuppressive antiinflammatory agent, a non-steroidal anti-inflammatory drug (NSAID), a salicylate antiinflammatory agent, a mineral; and a vitamins, such as vitamin A, vitamin B, vitamin C, vitamin D, vitamin E, and vitamin K. Said further therapeutic agent preferably comprises one or more of disease-modifying antirheumatic drugs (DMARDs), non-steroidal anti-inflammatories (NSAIDs), biological medicinal products and corticosteroids. NSAIDs include but are not limited to Cox-2 inhibitors, diclofenac, ibuprofen, naproxen, celecoxib, mefenamic acid, etoricoxib, indometacin and aspirin. Corticosteroids include but are not limited to triamcinolone, cortisone, prednisone, and methylprednisolone. Disease-modifying antirheumatic drugs (DMARDs), include but are not limited to auranofin, choroquine, cyclosporine, cyclophosphamide, gold preparations, hydroxychloroquine sulphate, leflunomide, methotrexate, penicillamine, sodium aurothiomalate and sulfasalazine.

In one embodiment the patients may additionally be treated with at least 2 or 3 DMARDs, preferably selected from the group consisting of hydroxychloroquine, leflunomide, methotrexate, minocycline, and sulfasalazine (e.g., methotrexate and hydroxychloroquine; methotrexate and leflunomide; methotrexate and sulfasalazine; sulfasalazine and hydroxychloroquine; methotrexate, hydroxychloroquine and sulfasalazine).

Optionally the patients may additionally be treated with biological medicinal products. Biological medicinal products, include but are not limited to selective costimulation modulators, TNF-α inhibitors, IL-1 inhibitors, IL-6 inhibitors and B-cell targeted therapies. Typically, the patient may additionally be treated with at least one biological treatment, for example a TNF-α inhibitor. Accordingly, in one aspect of the invention the patient may additionally be treated with at least one biological treatment which is a TNF-α inhibitor, typically Adalimumab (Humira), Certolizumab (Cimzia), Etanercept (Enbrel), Golimumab (Simponi), and/or Infliximab (Remicade).

In another embodiment, the further therapeutic agent may be a growth factor or other molecule that affects cell proliferation or activation. In a further embodiment that growth factor may induce final differentiation. In another embodiment, the growth factor may be a variant or fragment of a naturally- occurring growth factor. Methods of producing such variants are well known in the art, and may include, for example, making conservative amino acid changes, or by mutagenesis and assaying the resulting variant for the required functionality.

### PREPARATION METHODS OF THE PHARMACEUTICAL OR VETERINARY COMPOSITION OF THE INVENTION.

Methods for the isolation and culture of MSCs to provide eMSCs and cell populations of the invention, and compositions comprising cell populations of the invention are known in the art. Typically methods for the preparation of compositions comprising cell populations comprise the following steps:
(i) isolation of MSCs from tissue and selection by adherence to a suitable surface e.g. plastic
(ii) expansion of MSCs to provide cell populations of the invention comprising eMSCs.

The cell populations of the invention are suspended in the nutrient rich liquid preferably other than FBS prepared for cell culture, DMEM, used for the expansion of such cells or re-suspended in such media and cryopreserved during and/or subsequent to the expansion step by using DMSO.

### Isolation of MSC

MSCs for use in the invention may be isolated from any suitable tissue, such as but not limited to peripheral blood, bone marrow, placenta, adipose tissue, periosteum, dental pulp, spleen, pancreas, ligament, skin, tendon, skeletal muscle, umbilical cord and placenta. In a preferred embodiment, the MSCs are obtained from connective tissue, such as the stromal fraction of adipose tissue, hyaline cartilage, bone marrow, skin etc.

In one embodiment, the MSCs are from the stromal fraction of adipose tissue. In another embodiment, the MSCs are obtained from chondrocytes e.g. from hyaline cartilage. In another embodiment, the MSCs are obtained from skin. Also, in another embodiment, the MSCs are obtained from bone marrow.

The MSCs can be obtained from any suitable tissue and from any suitable animal, including humans. Typically, said cells are obtained from post-natal mammalian connective tissues.

The MSCs may also be isolated from any organism of the same or different species as the subject. Any organism with MSCs can be used with the invention. In one embodiment the organism may be mammalian such as a horse or a dog, and in another embodiment the organism is human.

Adipose-derived MSCs can be obtained by any means standard in the art. Typically said cells are obtained disassociating the cells from the source tissue (e.g. lipoaspirate or adipose tissue), typically by treating the tissue with a digestive enzyme such as collagenase. The digested tissue matter is then typically filtered through a filter of between about 20 micrometres (microns) to 1mm. The cells are then isolated (typically by centrifugation) and cultured on an adherent surface (typically tissue culture plates or flasks). Such methods are known in the art and e.g. as disclosed in U.S. Patent No. 6777231. According to this methodology, lipoaspirates are obtained from adipose tissue and the cells derived therefrom. In the course of this methodology, the cells may be washed to remove contaminating debris and red blood cells, preferably with PBS. The cells are digested with collagenase (e.g. at 37°C for 30 minutes, 0.075% collagenase; Type I, Invitrogen, Carlsbad, CA) in PBS. To eliminate remaining red blood cells, the digested sample can be washed (e.g. with 10% fetal bovine serum), treated with 160 mmol/L C1NH4, and finally suspended in DMEM complete medium (DMEM containing 10% FBS, 2 mmol/L glutamine and 1% penicillin/streptomycin). The cells can be filtered through a nylon mesh.

The cells are cultured in a suitable tissue culture vessel, comprising a surface suitable for the adherence of MSCs e.g. plastic. Non-adherent cells are removed e.g. by washing in a suitable buffer, to provide an isolated population of adherent stromal cells (e.g. MSC). Cells isolated in this way can be seeded (preferably 2-3xI0⁴ cells/cm²) onto tissue culture flasks and expanded at 37°C and 5% C0₂, changing the culture medium every 3-4 days. Cells are preferably passed to a new culture flask (1,000 cells/cm²) when cultures reach 90% of confluence.

Cell isolation is preferably carried out under sterile or GMP conditions.

In certain embodiments, the cells may be cultured for at least about 15 days, at least about 20 days, at least about 25 days, or at least about 30 days. Typically the expansion of cells in culture improves the homogeneity of the cell phenotype in the cell population, such that a substantially pure or homogenous population is obtained.

Cells are preferably detached from the adherent surface (e.g. by means of trypsin) and transferred to a new culture vessel (passaged) when cultures reach about 75%, 80%, 85%, 90% or 95% confluence.

In certain embodiments, the cells are passaged at least three times, which means that the cells are expanded in culture for at least three culture passages. In other embodiments, the cells are passaged at least four times, at least five times, at least six times, at least seven times, at least eight times, at least nine times, or at least ten times.

In certain embodiments, the cells are multiplied in culture for at least three population doublings. In certain embodiments, the cells are expanded in culture for at least four, five, six, seven, eight, nine, ten or 15 population doublings. In certain embodiments, the cells are expanded in culture for less than seven, eight, nine, ten or 15 population doublings. In certain embodiments, the cells are expanded in culture for between about 5 and 10 population doublings.

Cells may be cultured by any technique known in the art for the culturing of stromal stem cells. A discussion of various culture techniques, as well as their scale-up, may be found in Freshney, R.I., Culture of Animal Cells: A Manual of Basic Technique, 4th Edition, Wiley-Liss, 2000. Cells may be expanded using culture flasks or bioreactors suitable for large-scale expansion. Bioreactors suitable for the large-scale expansion of mesenchymal stromal cells are commercially available and may include both 2D (i.e. substantially planar) and 3D expansion bioreactors. Examples of such bioreactors include, but are not limited to, a plug flow bioreactor, a perfusion bioreactor, a continuous stirred tank bioreactor, a stationary-bed bioreactor.

In certain embodiments, the cells are cultured by monolayer culture. Any medium capable of supporting MSCs in tissue culture may be used. Media formulations that will support the growth of MSCs include, but are not limited to, Dulbecco's Modified Eagle's Medium (DMEM), alpha modified Minimal Essential Medium, and Roswell Park Memorial Institute Media 1640 (RPMI Media 1640). Typically, media useful in the methods of the invention may contain one or more compounds of interest, including, but not limited to antibiotics, mitogenic or differentiating compounds for stromal cells. The cells of the invention may be grown at temperatures between 31°C to 37°C in a humidified incubator. The carbon dioxide content may be maintained between 2% to 10% and the oxygen content may be maintained at between 1% and 22%.

Antibiotics which can be added to the medium include, but are not limited to penicillin and streptomycin. The concentration of penicillin in the chemically defined culture medium may be about 10 to about 200 units per ml. The concentration of streptomycin in the chemically defined culture medium may be about 10 to about 200 µg/ml.

Typically, the MSCs as used in the methods of the present invention are expanded cell populations, preferably said cells are expanded to provide a substantially pure or homogenous population.

It will be apparent to one skilled in the art that the method for preparation of the composition of the invention is not limiting, and that compositions of the invention prepared in any way are included within the scope of the invention.

It is important to note that during and/or subsequent to expansion, DMSO is added to the cell composition. Typically DMSO is added to the cells at passage 3, 4, 5, 6, 7, 8, 9, 10, 12, 15 or higher, e.g. DMSO may be added to the cells at passages 3-10 or 5- 10.

DMSO is usually added to cryopreserve the cell population of the invention. In this sense, the cell population of the invention is usually cryopreserved prior to administration, e.g. during and/or subsequent to expansion. Thus, the invention also provides cryopreserved cells of the invention. The cryopreservation method of the present invention requires the use of DMSO. Cells may be cryopreserved at any point during the isolation and/or expansion stages and thawed prior to administration. Typically cells may be cryopreserved at passage 3, 4, 5, 6, 7, 8, 9, 10, 12, 15 or higher, e.g. cells may be cryopreserved at passages 3-10 or 5- 10. Optionally the cells may be replated and cultured prior to administration.

In some embodiments, the cell populations of the invention in a composition of the invention may be pre-stimulated in order to enhance one or more of their proliferation capacity, migration capacity, survival capacity, therapeutic effect and immunoregulatory properties. In some embodiments, pre- stimulation may be achieved by contacting the MSCs with a cytokine. In some embodiments of the invention, pre-stimulation may be achieved by contacting the MSCs with IFN-γ.

In certain embodiments of the invention, the MSCs may be pre-stimulated using a concentration of IFN-y between 0.1 and 100 ng/ml. In further embodiments, the MSCs may be pre-stimulated using a concentration of IFN-y between 0.5 and 85 ng/ml, between 1 and 70 ng/ml, between 1.5 and 50 ng/ml, between 2.5 and 40 ng/ml, or between 3 and 30 ng/ml. Pre-stimulation may occur over a stimulation time longer than about 12 hours. Pre-stimulation may occur over a stimulation time longer than about 13 hours, longer than about 18 hours, longer than about 24 hours, longer than about 48 hours, or longer than about 72 hours.

In one embodiment, the MSCs of the invention may be stably or transiently transfected or transduced with a nucleic acid of interest using a plasmid, viral or alternative vector strategy. Nucleic acids of interest include, but are not limited to, those encoding gene products which enhance the production of extracellular matrix components found in the tissue type to be repaired, e.g. intestinal wall or vaginal wall.

The transduction of viral vectors carrying regulatory genes into the stromal cells can be performed with viral vectors, including but not limited to adenovirus, retrovirus or adenoassociated virus purified (e.g. by cesium chloride banding) at a multiplicity of infection (viral units / cell) of between about 10: 1 to 2000: 1. Cells may be exposed to the virus in serum free or serum-containing medium in the absence or presence of a cationic detergent such as polyethyleneimine or Lipofectamine^{™} for a period of about 1 hour to about 24 hours (Byk T. et al. (1998) Human Gene Therapy 9:2493-2502; Sommer B. et al. (1999) Calcif. Tissue Int. 64:45-49).

Other suitable methods for transferring vectors or plasmids into stromal cells include lipid/DNA complexes, such as those described in U.S. Pat. Nos. 5,578,475; 5,627, 175; 5,705,308; 5,744,335; 5,976,567; 6,020,202; and 6,051,429. Suitable reagents include lipofectamine, a 3: 1 (w/w) liposome formulation of the poly-cationic lipid 2,3-dioleyloxy-N-[2(sperminecarboxamido)ethyl]-N,N- dimethyl-I-propanaminium trifluoroacetate (DOSPA) (Chemical Abstracts Registry name: N-[2-(2,5-bis[(3-aminopropyl)amino]-I^{∼} oxpentyl} amino) ethyl]-N,N-dimethyl-2,3-bis(9-octadecenyloxy)-I-propanamin-ium trifluoroacetate), and the neutral lipid dioleoyl phosphatidylethanolamine (DOPE) in membrane filtered water. Exemplary is the formulation Lipofectamine 2000TM (available from Gibco/Life Technologies # 11668019). Other reagents include: FuGENETM 6 Transfection Reagent (a blend of lipids in non-liposomal form and other compounds in 80% ethanol, obtainable from Roche Diagnostics Corp. # 1814443); and LipoTAXITM transfection reagent (a lipid formulation from Invitrogen Corp., #204110). Transfection of stromal cells can be performed by electroporation, e.g., as described in M L. Roach and J.D. McNeish (2002) Methods in Mol. Biol. 185: 1. Suitable viral vector systems for producing stromal cells with stable genetic alterations may be based on adenoviruses and retroviruses, and may be prepared using commercially available virus components.

The transfection of plasmid vectors carrying regulatory genes into the MSCs can be achieved in monolayer cultures by the use of calcium phosphate DNA precipitation or cationic detergent methods (Lipofectamine^{™}, DOTAP) or in three dimensional cultures by the incorporation of the plasmid DNA vectors directly into the biocompatible polymer (Bonadio J. et al. (1999) Nat. Med. 5:753-759).

For the tracking and detection of functional proteins encoded by these genes, the viral or plasmid DNA vectors may contain a readily detectable marker gene, such as the green fluorescent protein or beta-galactosidase enzyme, both of which can be tracked by histochemical means.

Subsequent to expansion it is preferred that cell populations of the invention are assayed to determine the expression of characteristic markers to confirm their phenotype, which can be carried out by using conventional means.

The term "expressed" is used to describe the presence of a marker within a cell. In order to be considered as being expressed, a marker must be present at a detectable level. By "detectable level" is meant that the marker can be detected using one of the standard laboratory methodologies such as PCR, blotting or FACS analysis. The phenotypic surface marker characterization of a population of MSCs may be performed by any method known in the art. By way of example, but not limitation, this phenotypic characterization may be performed by individual cell staining. Such staining may be achieved through the use of antibodies. This may be direct staining, by using a labeled antibody or indirect staining, using a second labeled antibody against a primary antibody specific for the cell marker. Antibody binding may be detected by any method known in the art. Antibody binding may also be detected by flow cytometry, immunofluorescence microscopy or radiography.

Alternatively or additionally, a gene is considered to be expressed by a cell of the population of the invention if expression can be reasonably detected after 30 PCR cycles, which corresponds to an expression level in the cell of at least about 100 copies per cell. The terms "express" and "expression" have corresponding meanings. At an expression level below this threshold, a marker is considered not to be expressed. The comparison between the expression level of a marker in an adult stromal cell of the invention, and the expression level of the same marker in another cell, such as for example an embryonic stem cell, may preferably be conducted by comparing the two cell types that have been isolated from the same species. Preferably this species is a mammal, and more preferably this species is human. Such comparison may conveniently be conducted using a reverse transcriptase polymerase chain reaction (RT-PCR) experiment.

Cell-surface markers can be identified by any suitable conventional technique, usually based on a positive/negative selection; for example, monoclonal antibodies against cell-surface markers, whose presence/absence in the cells is to be confirmed, can be used; although other techniques can also be used.

The invention will now be further illustrated by the following examples. These examples are provided by way of illustration only, and are not intended to be limiting.

### EXAMPLES

### Example 1. Tissue extraction and MSC culture to provide the composition of the invention.

Adipose tissue was obtained from the different animals (dogs and horses) exemplified in examples 2 to 7, by aseptically collecting said tissue from the abdominal region of the animals by surgical excision.

MSCs were obtained as previously described Yanez, R., Lamana, M.L., García-Castro, J., Colmenero, I., Ramirez, M., Bueren, J.A., 2006. Adipose Tissue - Derived Mesenchymal Stem Cells Have In Vivo Immunosuppressive Properties Applicable for the Control of the Graft-Versus-Host Disease. Stem cells 24, 2582-2591. Briefly, adipose tissue was digested with collagenase IV (Serva Electrophoresis) at 0.2 U/fat mL over 1 h at 37°C. The resultant vascular stromal fraction was cultured in Dulbecco's Modified Eagle's medium (DMEM) and 1% Penicillin-Streptomycin-Glutamine (Biowest).

After 24 hours, cultures were washed with phosphate buffer saline (PBS) to remove non adherent cells and fresh medium was added. Cells were trypsinised when confluence was over 75% and sub-cultured until passage 3 was reached. Cultures were then trypsinised and frozen in DMEM with 10% dimethyl sulfoxide (DMSO) (v/v) (Sigma Aldrich). Before treatment, cells were defrosted without depleting the DMSO from the composition and sent to the clinic for use in the treatments stated in examples 2 to 7.

It is noted that the composition of the invention did not underwent a last culture passage just before release for treatment to eliminate the DMSO. In addition no manual methods of removing DMSO such as centrifugation, diffusion or a dilution-filtration method were used.

The defrosted compositions were prepared as prefilled syringes. Prefilled syringes for local administration had a final DMEM volume of between 0.5 and 3 ml and a cell concentration of between 1 x 10⁶ and 20 x 10⁶ cells in such volume. Prefilled syringes for local administration had a final DMEM volume of between 0.5 and 3 ml and comprised between about 0.25 x 10⁶ cells/kg to about 5 x 10⁶ cells/kg of subject weight.

### Example 2. Horse treated with the composition of the invention as defined in example 1 suffering from osteoarthritis.

The patient was a horseball sport horse, with a two years history of lameness that was controlled with corrective shoeing and pre-warming before the beginning of exercise. The animal is a 16 years old mare, diagnosed of degenerative joint disease and incipient osteoarthritis.

In the first visit the animal showed a lameness degree of 3 in a scale with 5 as maximum score, in the right forelimb, painful passive flexion, and increased lameness after a 1 minute forced flexion. In a radiological examination we confirmed the presence of osteophytes and exostosis in the dorsal area of the proximal interphalangeal joint.

A sample of adipose tissue was collected, and processed as described in example 1 for a period of 22 days. At the time of administration of stem cells, the mare showed the same symptoms as the first visit, and a total number of 10x10⁶ cells re-suspended in the described excipient and DMSO were administered via arthrocenthesis. 14 days after the administration, a general improvement was observed in the degree of lameness, decreasing from 3 to 1.

At 3 months after the administration, forced flexion of the distal phalanges of the right forelimb showed no pain, the horse shows no lameness at the beginning of the exercise or trot.

In the post-treatment radiological image, a stabilization of exostosis was shown in the second phalange on the back area of the joint. An improvement of the subchondral osteolysis in the second phalanx was also shown.

### Example 3. Horse treated with the composition of the invention as defined in example 1 suffering from a tendinal injuy.

The patient was an 8 years-old English thoroughbred gelding that in 2012 suddenly showed lameness after training. On examination, the animal was a grade 2/5 lame on the right forelimb. It showed edema in the superficial digital flexor tendon, and painful sensitivity to palpation.

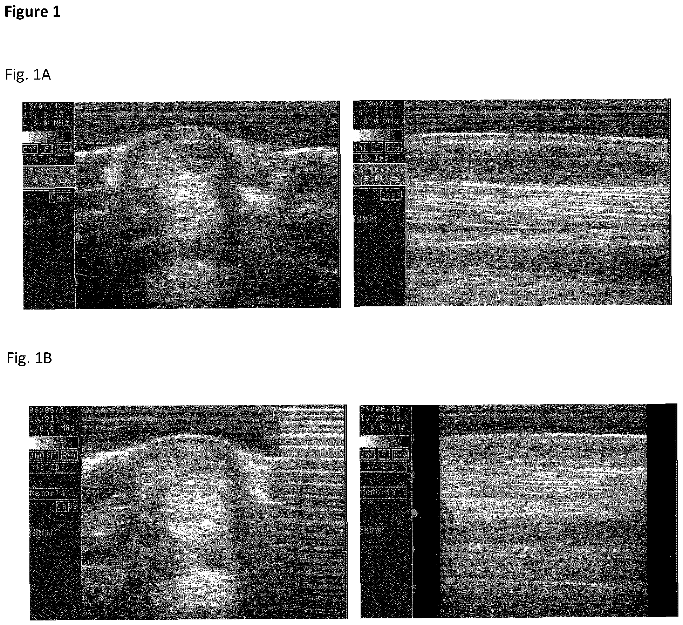

An ultrasound evaluation determined that significant injury had occurred to the mid-portion of the digital flexor tendon. There was a loss of echogenicity with lack of linear fiber pattern. The lesion occupied one third of the diameter of the tendon, with a near distant length of 5cm (Fig. 1A).

An adipose tissue sample was collected, and processed in the laboratory as previously indicated in example 1. In two weeks, the treatment was ready to use, and a new ultrasound evaluation was performed. The lesion persisted in the superficial digital flexor tendon, and decreased peritendinous inflammation was appreciated.

10x10⁶ MSCs were injected in an intra-tendinous way, in the area of the injury (ecoguided puncture in anechogenic area). At the 35 days re-check exam, there was no pain elicited on palpation and no lameness was detected. An ultrasound was performed and showed significant improvement in the appearance (see Fig. 1B). There was filling area of the lesion, as well as a more normal pattern of the fibers.

Rehabilitation program started at day 90 post-treatment, the patient was working again, earlier than recommended by the veterinary.

### Example 4. Horse treated with the composition of the invention as defined in example 1 suffering from chronic obstructive pulmonary disease or heaves.

Chronic obstructive pulmonary disease or Heaves in horses, is a chronic disorder of the airways similar to human asthma, which is characterized by variable and recurring airflow obstruction, bronchial hyperresponsiveness and airway inflammation. When stabled, heaves-susceptible horses develop a marked and persistent airway inflammation believed to be a consequence of aberrant innate and adaptive immunity responses. During disease exacerbation, horses present increased respiratory efforts at rest, coughing and exercise intolerance. Heaves is estimated to affect approximately 10-20% of adult horses in the northern hemisphere and other temperate climates.

No known cure for heaves already exists; so, susceptible horses recurrently develop airway obstruction and inflammation when exposed to offending environmental conditions. The standard management is thus organic dust avoidance, bronchodilators and corticosteroids.

The patient is an 11 years-old mixed-blood gelding that in 2007 started with respiratory problems in the upper airways, with cough and respiratory efforts at rest. The patient was diagnosed of obstructive mild pulmonary disease. We started therapy with bronchodilators which resolved the acute phase of the obstructive disorder. Yet, every year in winter a new exacerbation occurred, being necessary a new round of treatment.

In January 2011, the obstruction was more severe. General pulmonary bronchus was detected, coughing and exercise intolerance was described by the owner. The patient become refractory to the treatment in August of that year, so we decided to treat the animal with cellular therapy with Mesenchymal Stem Cells (MSC), which had shown positive effects in other animal models.

In February 2012 a total number of 1x10⁸ cells prepared as shown in example 1 were injected in an intravenous slow perfusion in a central vein. No adverse effects were detected. At the moment of infusion the constants were as follows: body temperature 36.6°C, negative pharyngeal reflex at cough, heart rate 36 beats per minute, breathing 18 breaths per minute. After 2 minutes hypercapnia: 54 beats/min, 36 breaths/min. Cough, and tracheal and bronchial rhonchus after hypercapnia. Rhonchus could be heard without exploration.

After 28 days, the exploration showed 36 beats/min, 12 breaths/min. After 2 minutes hypercapnia: 52 beats/min, 32 breaths/min. Rhonchus could be detected only under auscultation. The owner detects a general improve in the animal behavior.

Two months after the treatment, constants in rest were 25 beats/min and 12 breaths/min. The horse recovered strength and exercise tolerance. General exploration was normal.

### Example 5. Dog treated with the composition of the invention as defined in example 1 suffering from osteoarthritis secondary to Elbow Dysplasia.

The patient presented a history of chronic lameness in both fore legs, several months duration of refusal to go up steps, stiffness after rising from a laying position, and difficulty stretching. The dog also preferred lying to sitting. Previous diagnostic with radiographic images showed conformational abnormalities in both elbows. Pain was elicited upon flexion and extension of both articulations which manifested as vocalization and an attempt at biting the examiner.

Management of this problem had included exercise restriction and NSAID therapy and chondroprotective diet. The pain initially improved, but with time it returned with a significant lameness. Computer aided Tomography was performed and the patient was diagnosed of bilateral elbow dysplasia, fragmented coronoid and humeral Osteochondritis dissecans with synovial effusion. Left elbow (A - C) had a bone fragment (6x4x2mm, image A). Big size defect could also be seen (Image B), and several osteophytes in the articular margin (imagen C). On the left elbow (D - E) analogue changes could be seen but even more severe, with articular incongruence and more advanced osteoarthritis. Severe synovial effusion is shown in image F.

The dog underwent arthroscopy of the elbows, osteotomy in right elbow and a sample of adipose tissue collection in the abdominal area. Three weeks after the sample extraction, and using a sterile technique, each pre-loaded syringe with the invention was injected into each joint. The composition was prepared as illustrated in example 1 wherein DMSO was used and not depleted from the composition.

The injection sites were covered with sterile 3x3 gauze. These bandages were allowed to remain on the patient throughout the remainder of the day. Anti-inflammatory treatment every three days was maintained throughout the follow-up. The dog evolved favorably and 60 days post-treatment had improved, trotting lameness and increased the range of maximum extension of both elbows, not being painful to manipulation. The owner reported that the patient was more active, showed interest in playing, and attempted to go up the stairs.

A year later the dog appeared normal activity, running in their yard, playing with his ball, going up the stairs and sitting up on his hind legs to beg for a treat.

### Example 6. Dog treated with the composition of the invention as defined in example 1 suffering from osteoarthritis secondary to elbow and hip dysplasia and shoulder synovial capsule inflammation.

The patient exhibited a history of chronic lameness in the four extremities with extreme pain with more than a year of duration. Symptoms were very difficult to control with NSAID therapy.

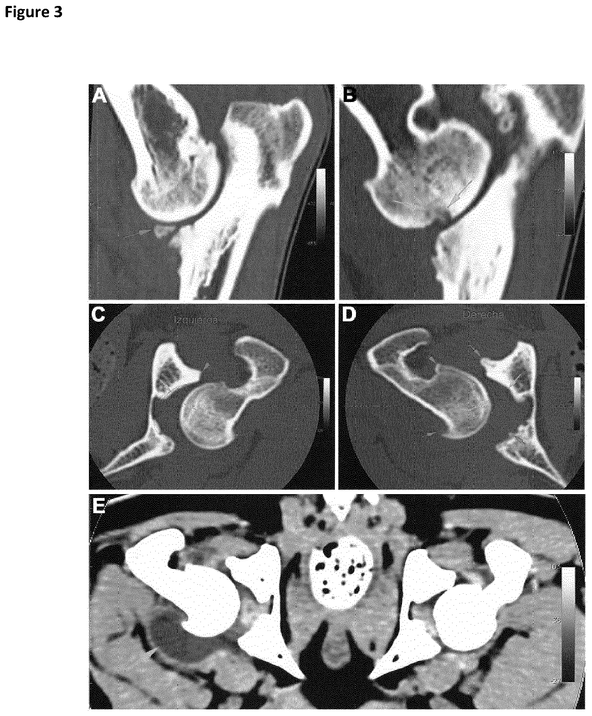

A CT scan was performed as shown in figure 3, and the diagnosis showed fragmented coronoid process and humeral cartilage injury in both elbows (A left and B right), thickened synovial capsules in both shoulders (C left and D right) and also hip dysplasia with edema (E).

The dog underwent arthroscopy in the left elbow for extraction of the coronoid fragments and osteotomy. The intervention was also employed to remove a sample of adipose tissue from the abdominal area and to manufacture the composition of the invention as illustrated in example 1.

A month after surgery, one dose of the invention was administrated in each of the six affected joints (hip, elbow and shoulder). Two months after the application of the cells the lameness was not noticeable, and no pain was detected in any joint exploration, only slightly at the maximum angles of manipulation in the right elbow.

### Example 7. Dog treated with the composition of the invention as defined in example 1 suffering

### from inflammatory bowel disease (IBD).

The patient was a female English Beagle, 16 months old, with 10 Kg of weight. The owners referred vomiting, diarrhoea, melaena, anorexia and weight loss in the first visit at the hospital.

Diagnosis of the disease comprised complete blood count (CBC), biochemistry, urinalysis, a faecal direct smear and zinc sulfate flotation for parasites, abdominal radiographs and ultrasound.

Gastroduodenoscopy and ileocolonoscopy was performed, with review of mucosal biopsy specimens, confirming the Inflamatory Bowel Disease diagnosis. Two accepted scores for this disease were calculated:
- CIBDAI (Clinical Inflammatory Bowel Disease Activity Index) that measures attitude and activity, appetite, vomiting, stool consistency, stool frequency, and weight loss; and
- CCECAI (Canine Chronic Enteropathy Clinical Activity Index): same as CIBDAI plus serum albumin concentration and the presence of ascites, peripheral oedema and pruritus.

The dog reached a score of 12 in both indexes.

The veterinary prescribed the standard treatment for these cases, i.e. elimination diet, corticosteroids, antibiotics, and antidiarrheal. After 8 months of symptoms duration, the therapy reported in this invention (example 1) was employed. A 3-week washout period eliminating all previous drugs was performed before entering the study. A preloaded syringe with the composition of the invention was diluted in 250 mL physiological saline serum and administered over 20 min through a peripheral IV cannula with a target dose of 2 × 10⁶ cells/kg bodyweight. The dog was monitored for 60 min following infusion and prior to being discharged.

After 2 weeks of MSCs therapy, a good clinical response was observed. Digestive symptoms disappeared (vomiting, diarrhoea, soft stools) and the animal increased activity and appetite and started gaining weight.

In haematology, and effective increasing serum albumin, cobalamin and folate was observed, and a decrease on C-Reactive values. A new gastroduodenoscopy and ileocolonoscopy was performed, observing macroscopic changes with a complete endoscopic remission and a partial histologic response. CIBDAI and CCECAI decreased to a value of 1.

### Example 7. Dog treated with the composition of the invention as defined in example 1 suffering from Atopic Dermatitis.

A 7 years old English Bull Dog was initially seen for a 4 year history of a poor dull dry scaly coat with pruritus. He was predominately an indoor dog and his pruritus was primarily over the paws and around mouth but did periodically rub or scratch at his chest and ears. Previously he had systemic glucocorticoids and desensibilization therapy but did not improve his quality of life. The dog had also been through an elimination diet trial for several weeks without results.

A complete haematology and biochemistry screen was performed, showing high levels of IgE. A Canine Atopic Dermatitis Extent and Severity Index (CADESI) was performed and scored at 35, and in a scratch score he reached 3 out of 5.

After diagnosis, the therapy reported in this invention (see example 1) was employed. A 3-week washout period eliminating all previous drugs was performed before entering the study. A preloaded syringe with the invention was diluted in 250 mL physiological saline serum and administered over 20 min through a peripheral IV cannula with a target dose of 2 × 10⁶ cells/kg bodyweight.

After 45 days of therapy, a good clinical response was observed. CADESI decreased to 15 and scratch reached a value of 0. At the time of this recheck the owner rated the therapy so positive and confirmed that no other supplements or topical therapy was being used except good skin hygiene.

### Example 8. Cell viability after thawing with DMSO.

The goal of cryopreservation is to maintain cell viability and functionality over time. This is achieved because the cold slows-down enzymatic reactions that occurred within the cells.

The critical period for cell survival during cryopreservation are the initial phased of freezing and the period of return to normal physiological conditions, so the thawing of stocks has to be done quickly, diluting the suspension and removing the preservative agent maximum speed. DMSO is used as a preservative for preventing crystals from forming during freezing and therefore enhances the viability of thawed cells. But when cells are thawed it must be removed promptly, according to the known literature, since this compounds is lethal to cells. Furthermore, although the health system ignores their therapeutic role, there are many items that support the therapeutic properties of DMSO. The aim of this example is to find out how long can MSCs in DMSO lasts without compromising its viability and how the media influences such viability.

The results are shown below:

### EXPERIMENT 1:

For this experiment we obtained 10X10⁶ cells of dog mesenchymal cells and we thawed one vial of MSCs in FBS with 10% (v/v) DMSO.

We then defrosted (at 37°C) the vial without depleting the DMSO from the composition. We counted the cells within a certain time period thus tracking the viability of cells over time
**Date:** 29/01/2016
**Sample:** D117 P5
**Preservation medium:** FBS with 10% DMSO

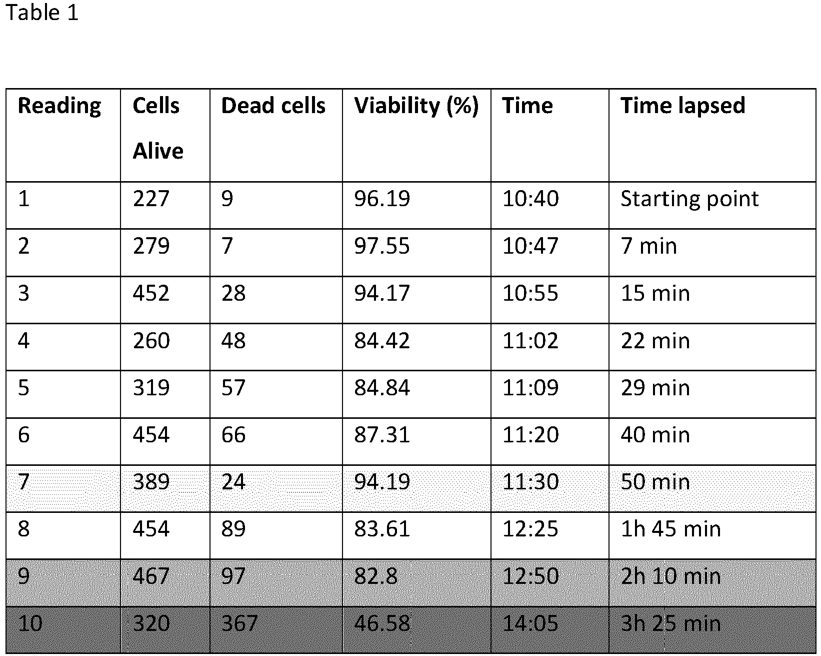

### EXPERIMENT 2:

2x10⁶ trypsinized canine MSCs were re-suspended in DMEM with 10% DMSO.
**Date:** 01/02/2016
**Sample:** D148 P1
**Preservation medium:** DMEM with 10% DMSO

**Table 2**

| **Reading** | **Cells Alive** | **Dead cells** | **Viability(%)** | **Time** | **Time lapsed** |
|---|---|---|---|---|---|
| 1 | 150 | 2 | 98.68 | 11:15 | Starting point |
| 2 | 170 | 1 | 99.42 | 11:45 | 30 min |
| 3 | 144 | 3 | 97.96 | 12:30 | 1h 15min |
| 4 | 150 | 1 | 99.34 | 12:45 | 1h 30min |
| 5 | 127 | 3 | 97.69 | 13:15 | 2h |
| 6 | 170 | 8 | 95.51 | 13:30 | 2h 15min |
| 7 | 160 | 3 | 98.16 | 14:00 | 2h 45min |
| 8 | 173 | 4 | 97.74 | 14:30 | 3h 15min |
| 9 | 148 | 9 | 94.27 | 15:00 | 3h 45min |
| 10 | 159 | 8 | 95.21 | 15:30 | 4h 15min |
| **OVERNIGHT** | | | | | |
| 11 | 109 | 14 | 81.95 | 8:45 | 21h 30min |
| 12 | 149 | 26 | 85.14 | 10:00 | 22h 45min |
| 13 | 163 | 19 | 89.56 | 11:00 | 23h 45min |
| 14 | 164 | 22 | 88.17 | 12:00 | 24h 45 min |
| 15 | 127 | 18 | 87.58 | 13:00 | 25h 45min |
| 16 | 129 | 17 | 88.36 | 14:00 | 26h 45min |

### EXPERIMENT 3:

For this experiment we obtained 2X10⁶ cells of dog mesenchymal stromal cells and we thawed one vial of MSCs in DMEM with 10% (v/v) DMSO.

We then defrosted (at 37°C) the vial without depleting the DMSO from the composition. We counted the cells within a certain time period thus tracking the viability of cells over time
**Date:** 01/02/2016 Frozen at -80°C
   02/02/2016 Defrosted
   03/02/2016 Readings until 25h 30min
**Sample:** D148 P1
**Preservation medium:** DMEM with 10% DMSO

**Table 3**

| **Reading** | **Cells Alive** | **Dead cells** | **Viability (%)** | **Time** | **Time lapsed** |
|---|---|---|---|---|---|
| 1 | 155 | 6 | 96.27 | 9:00 | Starting point |
| 2 | 166 | 7 | 95.95 | 9:30 | 30 MIN |
| 3 | 129 | 9 | 93.48 | 10:00 | 1H |
| 4 | 159 | 14 | 91.91 | 10:30 | 1h 30min |
| 5 | 150 | 5 | 96.77 | 11:00 | 2h |
| 6 | 130 | 8 | 94.2 | 11:30 | 2h 30min |
| 7 | 175 | 15 | 92.11 | 12:00 | 3h |
| 8 | 193 | 23 | 89.35 | 12:35 | 3h 35min |
| 9 | 153 | 11 | 93.29 | 13:00 | 4h |
| 10 | 140 | 9 | 93.96 | 13:30 | 4h 30min |
| 11 | 126 | 10 | 92.65 | 14.00 | 5h |
| 12 | 154 | 9 | 94.48 | 14:30 | 5h 30min |
| **overnight** | | | | | |
| 13 | 130 | 18 | 87.84 | 08:30 | 23h 30min |
| 14 | 132 | 17 | 88.59 | 9:30 | 24h 30min |
| 15 | 138 | 22 | 86.25 | 10:30 | 25h 30min |

## Claims

1. A pharmaceutical composition which comprises:
a. a population of mesenchymal stromal cells (MSCs) in which at least 50% of said population by number of cells are MSCs;
b. the basal media Dulbecco's Modified Eagle's medium (DMEM); and
c. dimethyl sulfoxide (DMSO) in a v/v concentration (DMSO volume percentage over the final volume of the nutrient rich liquid prepared for cell culture) of 10% ± 10%;
for use in the treatment of a subject suffering from osteoarthritis; wherein said composition is suitable for local administration and is administered via the intraarticular route.

2. The pharmaceutical composition for use according to claim 1, wherein the dimethyl sulfoxide (DMSO) is in a v/v concentration of 10%.

3. The pharmaceutical composition for use according to any of the precedent claims, wherein the mesenchymal stromal cells are umbilical-cord derived stromal cells, adipose tissue-derived stromal cells, expanded mesenchymal stromal cells, expanded adipose tissue-derived stromal cells, bone-marrow derived stromal cells or expanded bone-marrow derived stromal cells.

4. The pharmaceutical composition for use according to any of the precedent claims, wherein the MSCs are allogeneic or autologous.

5. The pharmaceutical composition for use according to any of the precedent claims, wherein the composition comprises between 1.0 x 10⁶ cells to 20 x 10⁶ cells.

6. A pharmaceutical composition as defined in any of claims 1 to 5, presented as a prefilled syringe or a cryotube or cryovial, for use according to claim 1.

7. The pharmaceutical composition for use according to claim 6, wherein the prefilled syringe comprises from 0.5 to 3 ml of DMEM.

8. The pharmaceutical composition for use according to any of claims 1 to 7, wherein the subject is a non-human mammal selected from the list consisting of a dog, a horse or a cat.

## Patentansprüche

1. Pharmazeutische Zusammensetzung umfassend:
a. eine Population von mesenchymalen Stromazellen (MSC), in welcher mindestens 50% der genannten Population in Anzahl von Zellen MSC sind;
b. das Basalmedium Dulbecco's Modified Eagle's medium (DMEM); und
c. Dimethylsulfoxid (DMSO) in einer Vol./Vol.-Konzentration (Vol.-% DMSO gegenüber des Endvolumens der nährstoffreichen Flüssigkeit, welche für die Zellkultur vorbereitet wird) von 10% ± 10%;
für deren Verwendung bei der Behandlung eines Individuums, welches unter Osteoarthritis leidet; wobei die genannte Zusammensetzung für die lokale Verabreichung geeignet ist und auf intraartikulärem Weg verabreicht wird.

2. Pharmazeutische Zusammensetzung für deren Verwendung nach Anspruch 1, wobei das Dimethylsulfoxid (DMSO) in einer Vol./Vol.-Konzentration von 10% ist.

3. Pharmazeutische Zusammensetzung für deren Verwendung nach einem der vorhergehenden Ansprüche, wobei die mesenchymalen Stromazellen aus Nabelschnur abgeleitete Stromazellen, aus Fettgewebe abgeleitete Stromazellen, expandierte mesenchymale Stromazellen, expandierte, aus Fettgewebe abgeleitete Stromazellen, aus Knochenmark abgeleitete Stromazellen oder expandierte, aus Knochenmark abgeleitete Stromazellen sind.

4. Pharmazeutische Zusammensetzung für deren Verwendung nach einem der vorhergehenden Ansprüche, wobei die MSC allogen oder autolog sind.

5. Pharmazeutische Zusammensetzung für deren Verwendung nach einem der vorhergehenden Ansprüche, wobei die Zusammensetzung zwischen 1,0 x 10⁶ Zellen und 20 x 10⁶ Zellen umfasst.

6. Pharmazeutische Zusammensetzung nach einem der Ansprüche 1 bis 5, dargeboten als Fertigspritze oder Kryoröhrchen oder Kryofläschchen, für deren Verwendung nach Anspruch 1.

7. Pharmazeutische Zusammensetzung für deren Verwendung nach Anspruch 6, wobei die Fertigspritze von 0,5 bis 3 ml DMEM umfasst.

8. Pharmazeutische Zusammensetzung für deren Verwendung nach einem der Ansprüche 1 bis 7, wobei das Individuum ein nichtmenschliches Säugetier ist, welches aus der Liste bestehend aus einem Hund, einem Pferd oder einer Katze ausgewählt wird.

## Revendications

1. Composition pharmaceutique qui comprend :
a. une population de cellules stromales mésenchymateuses (CSM) dans laquelle au moins 50% de ladite population en nombre de cellules sont des CSM ;
b. les milieux basaux moyen d'Eagle modifié par Dulbecco (DMEM) ; et
c. du sulfoxide de diméthyle (DMSO) dans une concentration v/v (volume en pourcentage de DMSO par rapport au volume final du liquide riche en nutriments préparé pour la culture cellulaire) de 10% ± 10% ;
pour son utilisation dans le traitement d'un sujet souffrant d'ostéoarthrite ; dans laquelle ladite composition est adéquate pour l'administration locale et est administrée par la voie intra-articulaire.

2. Composition pharmaceutique pour son utilisation selon la revendication 1, dans laquelle le sulfoxide de diméthyle (DMSO) est dans une concentration v/v de 10%.

3. Composition pharmaceutique pour son utilisation selon l'une quelconque des revendications précédentes, dans laquelle les cellules stromales mésenchymateuses sont des cellules stromales dérivées du cordon ombilical, des cellules stromales dérivées de tissu adipeux, des cellules stromales mésenchymateuses expansées, des cellules stromales dérivées de tissu adipeux expansées, des cellules stromales dérivées de moelle osseuse ou des cellules stromales dérivées moelle osseuse expansées.

4. Composition pharmaceutique pour son utilisation selon l'une quelconque des revendications précédentes, dans laquelle les CSM sont allogéniques ou autologues.

5. Composition pharmaceutique pour son utilisation selon l'une quelconque des revendications précédentes, dans laquelle la composition comprend entre 1,0 x 10⁶ cellules et 20 x 10⁶ cellules.

6. Composition pharmaceutique selon l'une quelconque des revendications 1 à 5, présentée comme seringue préchargée ou cryotube ou cryovial, pour son utilisation selon la revendication 1.

7. Composition pharmaceutique pour son utilisation selon la revendication 6, dans laquelle la seringue préchargée comprend de 0,5 jusqu'à 3 ml de DMEM.

8. Composition pharmaceutique pour son utilisation selon l'une quelconque des revendications 1 à 7, dans laquelle le sujet est un mammifère non humain choisi de la liste qui consiste en un chien, un cheval ou un chat.
